# EUROPEAN PATENT APPLICATION

(11) **EP 2 474 297 A1**
(43) Date of publication of application: **11.07.2012**
(21) Application number: 10813760.5
(22) Date of filing: 02.09.2010
(51) Int. Cl.: A61K 8/06, A61K 8/34, A61K 8/37, A61K 8/891, A61Q 1/12, A61Q 5/00, A61Q 19/00, A61Q 19/10

(54) **METHOD FOR PRODUCING O/W CREAM HAVING HIGH VISCOSITY**

(30) Priority: 04.09.2009 JP 2009205237
(71) Applicant: Shiseido Company, Ltd., Chuo-ku Tokyo 104-8010 (JP)
(72) Inventor: ARAKI Hidefumi, Yokohama-shi Kanagawa 224-8558 (JP); MIYAHARA Reiji, Yokohama-shi Kanagawa 224-8558 (JP)
(74) Representative: Henkel, Breuer & Partner
(86) International application number: PCT/JP2010/065004
(87) International publication number: WO 2011/027812

(57) **Abstract**

Disclosed is a convenient and economically advantageous method for producing an O/W cream having a high viscosity whereby an O/W cream having a high viscosity can be produced without adding a large amount of a thickener. This method for producing an O/W cream having a high viscosity is **characterized by** comprising: preparing an O/W emulsion (emulsified part) by emulsifying, at a temperature of 70°C or higher, an oily phase comprising a nonionic surfactant (A), a linear higher alcohol (B) that has 16 or more carbon atoms and can form, together with said nonionic surfactant, an α-gel in water, and an oily component (C), with an aqueous phase comprising water (D); cooling this emulsified part under stirring; and stopping the stirring at a temperature that is equal to or higher than the peak temperature in the temperature range, wherein the oily phase can form an α-gel in the aqueous phase, and lower than 70°C. The aforesaid peak temperature menus the exothermic peak temperature of the emulsified part in DSC. It is preferred that the viscosity of the O/W cream having a high viscosity is equal to or higher than 8,000 mPa·s (measured with a B-typc viscometer at 30°C).

## Description

### RELATED APPLICATIONS

This application claims the priority of Japanese Patent Application No. 2009-205237 filed on September 4, 2009, which is incorporated herein by reference.

### FIELD OF THE INVENTION

The present invention relates to a production method of a high-viscosity O/W cream, and in particular, relates to a production method that is excellent in simplicity and economy.

### BACKGROUND OF THE INVENTION

In the past, in order to produce a product such as a high-viscosity cream, it is necessary to use a higher alcohol at the content of 2 mass % or higher in the formulation to form an α-gel, or to use a large quantity of a thickener. As the preparation method of such a high-viscosity O/W cream, the following method has been used: not only components having a melting point at room temperature or higher, such as a nonionic surfactant, an aliphatic acid, a higher alcohol and a wax, but also all components to be blended are heated to the melting point or higher and emulsified; and the obtained emulsion is rapidly cooled with a cooling machine such as an Onlator to about room temperature (for example, refer to Non-patent Literature 1).

However, the use of a large of quantity of a thickener led to stickiness feeling when applied. When the conventional method, wherein the emulsion obtained by emulsification at the melting point or higher is cooled, is wasteful because of the energy necessary for heating and for the use of a heat exchanger. In addition, the environmental burden is high because a large amount of water is used for washing a cooling machine such as an Onlator after use. Accordingly, the development of a high-viscosity O/W cream that has comparable or superior usability to the conventional one and can be economically and easily produced without adding a large amount of a thickener and without using a conventional cooling apparatus such as an Onlator has been awaited.

### RELATED ART DOCUMENT

### NON-PATENT LITERATURE

Non-patent Literature 1: "Chemistry and Application of Surface Activity" (in Japanese) written by Manabu Senoo, Dainippon Tosho Co., Ltd., 1995; p 160.

### SUMMARY OF THE INVENTION

### PROBLEM TO BE SOLVED BY THE INVENTION

The present invention was made in view of the above-described situation of the background art, and the problem to be solved is to provide a simple and economical production method, without using a large amount of thickeners, of a high-viscosity O/W cream.

### MEANS TO SOLVE THE PROBLEM

In view of the above-described problems, the present inventors have diligently studied; as a result, the present inventors have found that a high-viscosity O/W cream can be produced by: emulsifying, at 70°C or higher, an oil phase comprising components such as a nonionic surfactant, a linear higher alcohol that has 16 or more carbon atoms and can form an α-gel in water with the nonionic surfactant, an oil component, with a water phase component such as water and moisturizer; cooling the obtained emulsion while being stirred; and stopping the stirring at the peak temperature or higher in the temperature range wherein the oil phase forms an α-gel in the water phase. This finding has led to completion of the present invention.

That is, the present invention is a production method of a high-viscosity O/W cream comprising the steps of :
emulsifying, at 70 °C or higher, an oil phase with a water phase to prepare an emulsified part that is an O/W emulsion, wherein
   the oil phase comprises
      (A) a nonionic surfactant,
      (B) a linear higher alcohol that has 16 or more carbon atoms and can form an α-gel in water with the nonionic surfactant, and
      (C) an oil component, and
   the water phase comprises
      (D) water;
cooling the emulsified part while the emulsified part is being stirred; and
stopping the stirring at the peak temperature or higher in a temperature range wherein the oil phase forms an α-gel in the water phase, but lower than 70°C.

It is preferable in the production method of the high-viscosity O/W cream that the method comprises the steps of:
emulsifying, at 70°C or higher, the oil phase with a portion of the water phase to prepare the emulsified part,
mixing the remaining water phase, which is a main water phase and at 20 to 40°C. with the emulsified part while the emulsified part is being stirred, to cool the emulsified part; and
stopping the stirring at the peak temperature or higher in the temperature range wherein the oil phase forms the α-gel in the water phase, but lower than 70 °C.

It is preferable in the production method of the high-viscosity O/W cream that the temperature when the stirring is stopped is 55 °C or lower.
It is preferable in the production method of the high-viscosity O/W cream that the peak temperature in the temperature range wherein the oil phase forms the α-gel in the water phase is the exothermic peak, which is measured by differential scanning calorimetry DSC, of the emulsified part.

It is preferable in the production method of the high-viscosity O/W cream that the viscosity of the high-viscosity O/W cream measured with a B-type viscometer at 30 °C is 8,000 mPa·s or higher.
It is preferable in the production method of the high-viscosity O/W cream that the mass ratio of the main water phase and the emulsified part is 3:1 to 1:1.

### EFFECT OF THE INVENTION

According to the production method of the O/W emulsion composition of the present invention, a high-viscosity O/W cream can be easily produced with low energy without adding a large amount of a thickener and without using a cooling machine such as an Onlator; thus it is very economical. In addition, according to the production method of the present invention, a more viscous cosmetic can be obtained even when the formulation is the same as that for a low-viscosity cosmetic; therefore, the use of a large amount of thickener can be suppressed, leading to an improved texture in use.

### BRIEF DESCRIPTION OF THE DRAWINGS

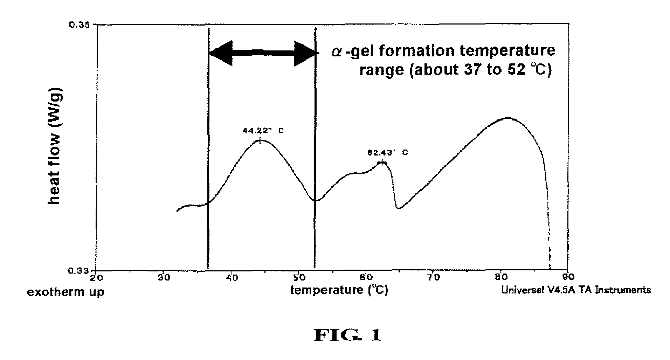

Fig 1 shows a result for the DSC measurement of an emulsified part for a high-viscosity O/W cream of the present invention.

### MODE FOR CARRYING OUT THE INVENTION

Hereinafter, the constitution of the present invention will be described in detail. The high-viscosity O/W cream of the present invention can be obtained by:
emulsifying with mixing, at 70 °C or higher, an oil phase with a water phase to prepare an emulsified part that is an O/W emulsion, wherein
   the oil phase comprises
      (A) a nonionic surfactant,
      (B) a linear higher alcohol that has 16 or more carbon atoms and can form an α-gel in water with the nonionic surfactant, and
      (C) an oil component, and
   the water phase comprises
      (D) water;
cooling the emulsified part while the emulsified part is being stirred; and
stopping the stirring at the peak temperature or higher in a temperature range wherein the oil phase forms an α-gel in the water phase, but lower than 70 °C, during the cooling. The high-viscosity O/W cream is **characterized in that** an α-gel is formed in the interface of the emulsion particles of the O/W cream.

The α-gel is an associated complex consisting of lamellar bimolecular membranes, which are formed by a surfactant with a linear higher alcohol having 16 or more carbon atoms or a non-neutralized fatty acid in the presence of water. This α-gel formation temperature varies depending upon the chain length of the higher alcohol or fatty acid and the mole ratio between the higher alcohol or non-neutralized fatty acid and the surfactant. This is described in "Physical Chemistry of Cetyl Alcohol" (in Japanese) (Fragrance Journal Ltd., 1992) written by Shoji Fukushima.

(A) A nonionic surfactant used in the present invention is not limited in particular so far as the oil phase can form an α-gel, in the water phase, with the nonionic surfactant.

Examples of (A) the nonionic surfactant used in the present invention include polyoxyethylene glycerol fatty acid esters, polyoxyethylene methylpolysiloxane copolymer, polyoxyethylene sorbitan fatty acid esters, polyoxyethylene alkyl ethers, maltitol hydroxy aliphatic alkyl ethers, alkylated polysaccharides, alkyl glucosides, sucrose fatty acid esters, and polyoxyethylene hydrogenated castor oil glyceryl. Hydrophilic surfactants are preferable, and in particular, those with the HLB of 8 or higher are preferable. However, a lipophilic nonionic surfactant such as glyceryl monostearate can be used in combination therewith.

(B) A linear higher alcohol having 16 or more carbon atoms used in the present invention is not limited in particular so far as it can form an α-gel with a nonionic surfactant in water and it can be used in the fields of cosmetics, pharmaceuticals, quasi-drugs, etc. Examples thereof include cetyl alcohol, stearyl alcohol, and behenyl alcohol. Preferably, it is a linear higher alcohol having 16 to 24 carbon atoms.
For the formation of α-gel, it is preferable to add batyl alcohol, monoglycerides, etc.

The concentrations of (A) a nonionic surfactant and (B) a linear higher alcohol having 16 or more carbon atoms are not limited in particular. Preferably, the total amount of (A) the nonionic surfactant and (B) the linear higher alcohol having 16 or more carbon atoms, in the high-viscosity O/W cream, is 0.5 to 10 parts by mass with respect to 10 parts by mass of (C) the oil component. If the total amount is less than 0.5 parts by mass, a high-viscosity O/W cream having high stability may not be obtained because of low surfactant content. If the total amount exceeds 10 parts by mass, there is an unfavorable trend from the standpoint of usability because of too much surfactant.

(C) An oil component used in the present invention is not limited in particular. Examples thereof include the followings:
Liquid fats such as avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, china paulownia oil, Japanese paulownia oil, jojoba oil, germ oil and triglycerides;
Solid fats such as cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neat's-foot oil, Japan wax, and hydrogenated castor oil;

Waxes such as beeswax, candelilla wax, cotton wax, carnauba wax, bayberry wax, ibota wax, spermaceti wax, montan wax, rice bran wax, lanolin, kapok wax, acetylated lanolin, liquid lanolin, sugar cane wax, lanolin fatty acid isopropyl ester, hexyl laurate, hydrogenated lanolin, jojoba wax, lanolin wax, shellac wax, POE lanolin alcohol ether, POE lanolin alcohol acetate, POE cholesterol ether, lanolin fatty acid polyethylene glycol ester, and POE hydrogenated lanolin alcohol ether;

Hydrocarbon oils such as liquid paraffin, ozokerite, squalane, pristane, paraffin, ceresin, squalene, polybutene, and microcrystalline wax;

Synthetic ester oils such as isopropyl myristate, cetyl octanoate, octyldodecyl myristate, isopropyl palmitate, butyl stearate, hexyl laurate, myristyl myristate, decyl oleate, hexyldecyl dimethyloctanate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, monoisostearate N-alkylglycol, neopentylglycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptlyundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate; and

Silicone oils such as linear polysiloxanes (e.g., dimethylpolysiloxane, methylphenylpolysiloxane, diphenylpolysiloxane, etc.), cyclic polysiloxanes (e.g., octamethylcyclotetrasiloxane, decamethylcyclopentasiloxane, odecamethylcyclohexasiloxane, etc.), silicone resins having a three-dimensional network structure, silicone rubbers, various modified polysiloxanes (e.g., amino-modified polysiloxane, polyether-modified polysiloxane, alkyl-modified polysiloxane, fluorine-modified polysiloxane, etc.) and acrylsilicone.

In addition, it is preferable that one or more higher fatty acids are blended in (C) the oil component. By blending the higher fatty acid these in the oil component, the emulsion particles become much finer, and a more stable O/W cream can be obtained. As the higher fatty acid, those having 16 to 24 carbon atoms are preferable. Examples thereof include unsaturated fatty acids such as oleic acid, linoleic acid, and linolenic acid; isostearic acid, isopalmitic acid, isomyristic acid, behenic acid, stearic acid, palmitic acid, and myristic acid. The content of higher fatty acids in the O/W cream is preferably 0.1 to 3 mass % and more preferably 0.2 to 1.5 mass %.

The water phase, comprising (D) water, used in the present invention is not limited in particular so far as the main medium of the water phase is water, or water with an aqueous solvent. In addition to water or an aqueous solvent, the components normally used in cosmetics, pharmaceuticals, etc. may be blended thereto within the quantity range that does not affect the stability
The total blending quantity of water in the high-viscosity O/W cream of the present invention is not limited in particular. It is generally preferable that the blending quantity is 40 to 95 weight % of the total amount of the high-viscosity O/W cream.

It is preferable in the production method of the present invention that the water phase comprises an aqueous solvent. The aqueous solvent is not limited in particular and suitably selected from publicly known aqueous solvents depending upon the types of (C) an oil component and (A) a nonionic surfactant. Here, the aqueous solvent means a substance that is liquid at room temperature and water-soluble. Examples thereof include polypropylene glycol polyethylene glycol copolymer or its dimethyl ether, polyethylene glycol or its alkyl ethers, dipropylene glycol, isoprene glycol, and propylene glycol.

In the combination of (C) an oil component and an aqueous solvent used in the present invention, it is necessary that the aqueous solvent is immiscible with the oil component. Examples of such combinations include the followings: when (C) the liquid oil component is dimethylpolysiloxane, the aqueous solvent is polypropylene glycol polyethylene glycol copolymer or its dimethyl ether, polyethylene glycol or its ethyl ether, 1,3-butylene glycol, dipropylene glycol, isoprene glycol, etc.; when (C) is cyclodimethicone (pentamer), the aqueous solvent is polypropylene glycol polyethylene glycol copolymer or its dimethyl ether, polyethylene glycol or its ethyl ether, etc.; when (C) is methylphenylpolysiloxane, the aqueous solvent is polypropylene glycol polyethylene glycol copolymer or its dimethyl ether, 1,3-butylene glycol, glycerin, etc.; when (C) is liquid paraffin, the aqueous solvent is polypropylene glycol polyethylene glycol copolymer or its dimethyl ether, polyethylene glycol or its ethyl ether, 1,3-butylene glycol, dipropylene glycol, isoprene glycol, etc.

As specific examples of the aqueous solvent used in the present invention, the aqueous solvents that have zero to three hydroxyl groups in the molecule can be listed. More specific examples include polypropylene glycol polyethylene glycol copolymer or its alkyl ethers, polyethylene glycol or its alkyl ethers, polyoxyalkylene dicarboxylates, 1,3-butylene glycol, dipropylene glycol, isoprene glycol, 1,2-pentane glycol, 1,2-hexane glycol, 2-methyl-1,3-propanol, ethyl carbitol, 1,2-butylene glycol, glycerin, etc. The aqueous solvent can be suitably selected and used from these solvents depending upon the types of (C) an oil component and (A) a nonionic surfactant. As the aqueous solvent used in the present invention, two or more of these solvents can be used in combination.
On the other hand, the water-soluble materials that have four or more hydroxyl groups in the molecule usually become solid at room temperature, and cannot often achieve the effect of the aqueous solvent in the present invention.

The blending quantity of the aqueous solvent used in the present invention is not limited in particular. Preferably, it is 5 mass % or higher of the O/W emulsified part prepared at 70 °C or higher. If the blending quantity is less than 5 mass %, the preparation of a stable high-viscosity O/W cream tends to be difficult.

In the production method of the present invention, when the water phase is separated into a water phase to be used for the emulsification with the oil phase (i.e., a portion of the water phase) and a water phase to be used for the mixing with the emulsified part (i.e., main water phase), it is preferable that the mass ratio of the main water phase and the emulsified part is 3:1 to 1:1. If the mass ratio departs from the above range, the high-viscosity O/W cream may not be obtained or the usability may be inferior.

The α-gel formation temperature range in the production method of the present invention depends on the types of blended surfactant and higher alcohol, the constitution of the water phase, etc.; however, it is typically about 37 to 52 °C.

The temperature of the main water phase, which is added later, is adjusted so that the temperature immediately after the completion of mixing of the main water phase with the emulsified part is preferably within the temperature range of α-gel formation (for example, 37 to 52 °C), and more preferably it is the peak temperature or higher within the temperature range. Specifically, it is preferable that the temperature of the main water phase is 20 to 40 °C. If the temperature is lower than 20 °C, the energy necessary to cool the main water phase becomes excessive, and it tends to be less economical. Or, the temperature immediately after the completion of mixing of the main water phase becomes too low, and the intended high-viscosity O/W cream may not be obtained. If the temperature exceeds 40 °C, there will be little merit from the energy standpoint.

It can be confirmed by DSC (differential scanning calorimetry) that the oil phase forms an α-gel in the water phase. The emulsified part obtained by emulsifying the oil phase comprising (A) a hydrophilic nonionic surfactant, (B) a linear higher alcohol having 16 or more carbon atoms, and (C) an oil component in the water phase is measured by DSC (differential scanning calorimetry) while lowering the temperature to 70 °C or lower. Then an exothermic peak is observed, and this indicates that the oil phase has formed an α-gel in the water phase.
"The α-gel formation temperature range" in the present invention means the temperature range of the exothermic peak due to the formation of α-gel. The peak temperature means the temperature at the top of the exothermic peak (refer to Fig. 1). In the DSC measurement, other exothermic peaks may be detected in addition to the exothermic peak due to the formation of α-gel. These are exothermic peaks due to simple solidification of components contained in the emulsified part. The exothermic peak due to the formation of α-gel is different from these exothermic peaks due to solidification.

The peak temperature of the exothermic peak measured by DSC (differential scanning calorimetry) is considered to correspond to the melting point of the emulsified part in the production method of the present invention. In the production method of the present invention, by stopping the stirring at the peak temperature or higher in the temperature range of α-gel formation, the intended high-viscosity O/W cream can be obtained.

The O/W cream prepared by the production method of the present invention is in a state that an associated complex consisting of lamellar bimolecular membranes formed from a nonionic surfactant and a higher alcohol, namely α-gel, is present in the interface of emulsion particles formed from a nonionic surfactant, a higher alcohol, and an oil component in the presence of water.

In the present invention, "cream" is defined to have a viscosity to the extent that it does not flow when the composition is filled in a glass bottle etc. and the bottle is tilted. Specifically, the viscosity is preferably 8,000 mPa·s or higher and more preferably 12,000 mPa·s or higher with a B-type viscometer (rotor: No. 3, rotation speed: 12 rpm). The upper limit of viscosity is not limited in particular; however, if the viscosity is too high, the usability may become poor. Thus, it is preferable that the viscosity is about 100,000 mPa-s (30 °C) or lower.

Hereinafter, the concept for the production method of the high-viscosity O/W cream of the present invention will be explained.

### Concept:

(1) An oil phase comprising (A) a nonionic surfactant, (B) a linear higher alcohol that has 16 or more carbon atoms and can form an α-gel in water with the nonionic surfactant, and (C) an oil component is emulsified, at 70 °C or higher, with a water phase comprising (D) water, and the obtained emulsified part is cooled while being stirred. On this occasion, the stirring is stopped at the peak temperature or higher in the temperature range wherein the oil phase forms an α-gel in the water phase. For example, the oil phase and the water phase are emulsified at 70 °C or higher (preferably 70 to 80 °C, and more preferably 70 to 75 °C), the obtained emulsified part, under stirring, is left to cool or cooled with any cooling apparatus, the stirring is stopped when the temperature of the emulsified part, during cooling, becomes the peak temperature or higher, in the temperature range of α-gel formation, and then the emulsified part is left to cool. The apparatuses used in emulsification, stirring, and cooling can be suitably selected from normally used apparatuses. The thus far used apparatuses can be applied, and no special equipment is necessary.
According to the above method, even when the formulation with which only low-viscosity O/W emulsion such as milky lotion is obtained by the conventional method is used, a high-viscosity cream can be obtained.

(2) In the above-described method, however, a considerable amount of time may be necessary if the cooling to the vicinity of the peak temperature in the temperature range is carried out by being left to cool. If a cooling apparatus such as an Onlator is used, it is economically disadvantageous. Thus, in order to solve the above-described problem, a highly concentrated emulsified part is prepared at 70 °C or higher with the use of a part of the water phase. Subsequently, the remaining water phase (main water phase) at 20 to 40 °C is gradually mixed to the highly concentrated emulsified part until the temperature range of α-gel formation is reached. By mixing of this main water phase, the high-temperature emulsified part is cooled as well as diluted. And, when the stirring is stopped at the peak temperature or higher in the temperature range of α-gel formation, a high-viscosity O/W cream is efficiently prepared.

In the production method of the present invention, the stirring is terminated at the peak temperature or higher, in the temperature range of α-gel formation, and lower than 70 °C. When the merit of low-energy production is considered, the temperature when the stirring is terminated is preferably the peak temperature or higher and 55 °C or lower, and more preferably 50°C. or lower. When the main water phase is mixed with the emulsified part, the mixing of the main water phase is preferably completed within the temperature range of α-gel formation, and more preferably within the temperature range of α-gel formation and at the peak temperature or higher. The mixing can also be completed at the same time as the stopping time of stirring.

The high-viscosity O/W cream obtained by the above-described production method can stably exist over a wide temperature range for a long period of time though the particle size is small, ranging from 1 to 3 µm.

In the conventional production method of a high-viscosity O/W cream, the following method has been used: water, a moisturizer, and a thickener are dissolved and heated to about 70 °C to prepare a water phase in advance; an oil phase obtained by uniform mixing an oil component, a higher alcohol, and a hydrophilic nonionic surfactant, at about 70 °C, is emulsified into the water phase while being stirred with a homogenizer; and the obtained emulsion is rapidly cooled with an Onlator to about 35 °C. However, the energy use in the conventional production method is wasteful because of heating and the use of a heat exchanger, and the consumption of water used for the cooling machine is large; thus the environmental burden has been high.

On the other hand, the high-viscosity O/W cream obtained by the production method of the present invention can be easily produced with low energy because a cooling machine such as an Onlator is not used when emulsification, and it is not necessary to heat a large amount of water or an aqueous solvent. In addition, the emulsification is virtually carried out only with a nonionic surfactant, whose irritation to the human body is relatively small; thus it has excellent safety.

In past, a high-viscosity O/W cream has been produced with the use of a large amount of materials having viscosity-increasing effect, such as thickeners, higher alcohols, and aliphatic acids. The production method of the present invention, however, since the viscosity can be increased by the control of the temperature when the stirring is stopped during the cooling and such a large amount of the thickeners and the like described above is not used, a high-viscosity O/W cream having a good feeling of use can be produced.

Furthermore, in the production method of the present invention, the preparation temperature can be controlled only by adding the water phase at 20 to 40 °C to the emulsified part produced in advance at 70 to 80 °C, and a high-viscosity O/W cream can be obtained. Thus, the conventional production process can be drastically simplified.

The high-viscosity O/W cream of the present invention can be preferably applied to external preparations used on the body such as the skin and hair in the fields such as cosmetics, pharmaceuticals, and quasi-drugs. For example, it can be used for products such as skin cosmetics, hair cleanser, skin cleanser and hair dressing.
In the high-viscosity O/W cream of the present invention, in addition to the above-described essential components, the components normally used in cosmetics, pharmaceuticals, etc. can be blended within the range that the effect of the present invention, such as stability, is not undermined. Examples of such components include the following:

Moisturizers such as polyethylene glycol and its alkyl ethers, glycerin, sorbitol, xylitol, maltitol, mucopolysaccharides, hyaluronic acid, chondroitin sulfate, and chitosan. Thickeners such as methylcellulose, ethylcellulose, gum arabic, and polyvinyl alcohol. Organic solvents such as ethanol. Antioxidants such as butylhydroxytoluene, tocopherol, and phytic acid. Antimicrobial preservative agents such as benzoic acid, salicylic acid, sorbic acid, paraoxybenzoic acid ester (ethylparaben, butylparaben, etc.), and hexachlorophene. Amino acids such as glycine, alanine, valine, leucine, serine, threonine, phenylalanine, tyrosine, aspartic acid, asparagine, glutamine, taurine, arginine, and histidine; and their salts. Organic acids such as acyl sarcosinic acid (for example, sodium lauroyl sarcosinate), glutathione, citric acid, malic acid, tartaric acid, and lactic acid.

Vitamin A and its derivatives; vitamin Bs such as vitamin B₆ hydrochloride, vitamin B₆ tripalmitate, vitamin B₆ dioctanoate, vitamin B₂ and its derivatives, vitamin B₁₂, and vitamin B₁₅ and its derivatives; vitamin Cs such as ascorbic acid, ascorbyl phosphate ester (its salts), and ascorbyl dipalmitate; vitamin Es such as α-tocopherol, β-tocopherol, γ-tocopherol, vitamin E acetate, and vitamin E nicotinate; vitamin Ds; and vitamins such as vitamin H, pantothenic acid, and pantethine. Various agents such as nicotinic acid amide, benzyl nicotinate, γ-oryzanol, allantoin, glycyrrhizic acid (its salts), glycyrrhetinic acid and its derivatives, hinokitiol, mucidin, bisabolol, eucalyptol, thymol, inositol, saponins (saikosaponin, ginseng saponin, luffa cylindrica saponin, sapindus mukorossi saponin, etc.), pantothenyl ethyl ether, ethinylestradiol, tranexamic acid, cepharanthine, and placenta extract.

Natural extracts obtained by the extraction with a solvent such as organic solvents, alcohols, polyhydric alcohols, water, aqueous alcohols, from materials such as sorrel, Sophora flavescens Aiton, cow lily, orange, sage, thyme, yarrow, mallow, cnidium root, Swertia japonica, Angelica acutiloba, spruce, birch, field horsetail, Luffa cylindrica, horse chestnut, saxifrage, arnica, lily, mugwort, peony, aloe, gardenia, and sawara cypress. Cationic surfactants such as stearyltrimethylammonium chloride, benzalkonium chloride, and laurylamine oxide. Sequestering agents such as disodium edetate, trisodium edetate, sodium citrate, sodium polyphosphate, sodium metaphosphate, and gluconic acid. Neutralizing agents such as potassium hydroxide, sodium hydroxide, and triethanolamine.
In addition, Perfumes, scrubbing agents, powder, coloring agents, whitening agents, UV protection agents such as UV absorbers and UV scattering agents, etc. may be appropriately used so far as effects such as stability is not impaired.

### EXAMPLES

Hereinafter, the present invention will be explained in further detail with reference to the examples of high-viscosity O/W creams of the present invention. However, the present invention is not limited by these examples. Unless otherwise noted, the blending quantities are all expressed in mass %.

Initially, the methods for the test and the evaluation for each production example will be explained.

### (Viscosity)

With the use of a B-type viscometer (rotor: No. 4, rotation speed: 12 rpm), the viscosity at 30 °C was measured.

### (Thickening effect)

Based on the increasing rate of viscosity compared with the viscosity of O/W emulsion compositions prepared by a similar method to that of Comparative Example 1 (conventional process) with the use of the same formulation, the judgment was carried out as follows.
○: 6 times or higher
○Δ: 4 times or higher
Δ: 2 times or higher
x: less than 2 times
Here, Δ or higher was evaluated to be acceptable.

### (Stability)

The prepared O/W cream was stored in a constant temperature bath, at a low temperature (-10 °C) to a high temperature (60 °C), for 1 month. Then, the emulsion state was observed with the naked eye to check for the destruction or coalescence of emulsion particles and the judgment was carried out as follows.
○: The destruction and coalescence of emulsion particles are not observed.
Δ: The destruction or coalescence of emulsion particles is partially observed.
x: The destruction or coalescence of emulsion particles is observed.
Here, Δ or higher was evaluated to be acceptable.

### (Evaluation of usability)

The usability (spreadability, fit feeling, stickiness, refreshing feeling, and moist feeling) was evaluated by 10 panelists. Based on the number of panelists who answered that the product was comprehensively superior to the conventional product (i.e., the high-viscosity formulation of the Comparative Example 6 in the table below), the judgment was carried out as follows.
○ : 9 panelists or more and 10 panelists or less
○Δ: 7 panelists or more and 8 panelists or less
Δ: 5 panelists or more and 6 panelists or less
Δx: 3 panelists or more and 4 panelists or less
x : 2 panelists or less
Here, Δ or higher was evaluated to be acceptable as it had superiority.

In the table below, the evaluation results of viscosity, stability, and the usability for the O/W emulsion compositions produced by various production methods, and the blending formulation thereof are summarized. The quantities in the table below are expressed in mass %.

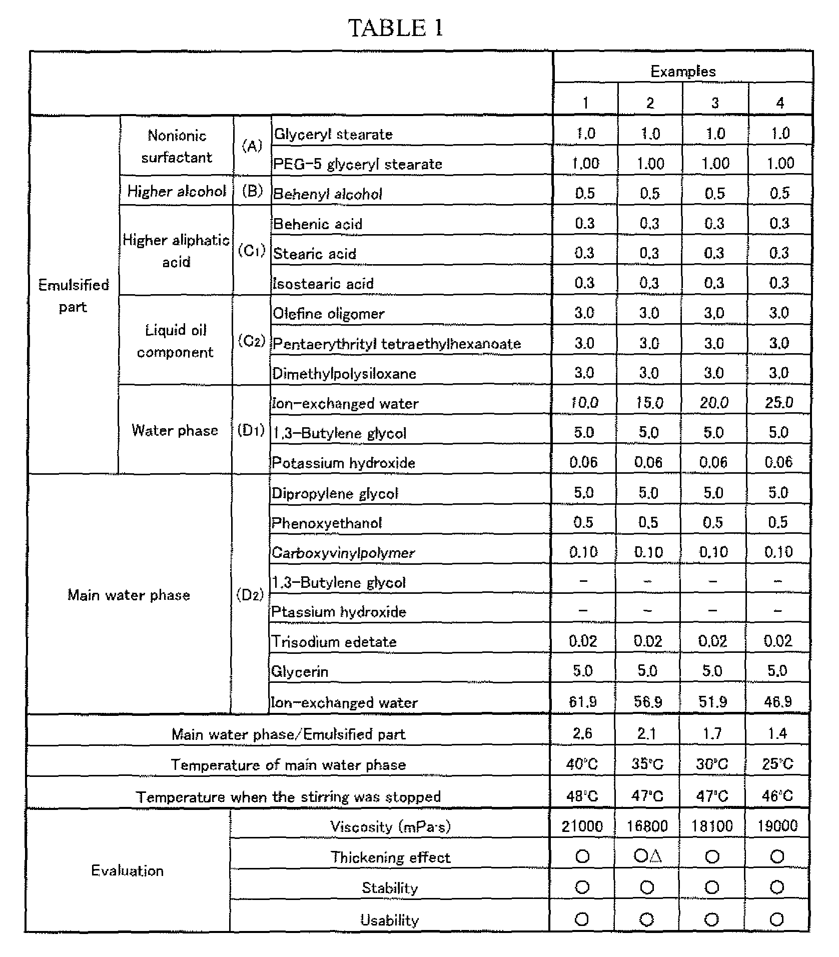

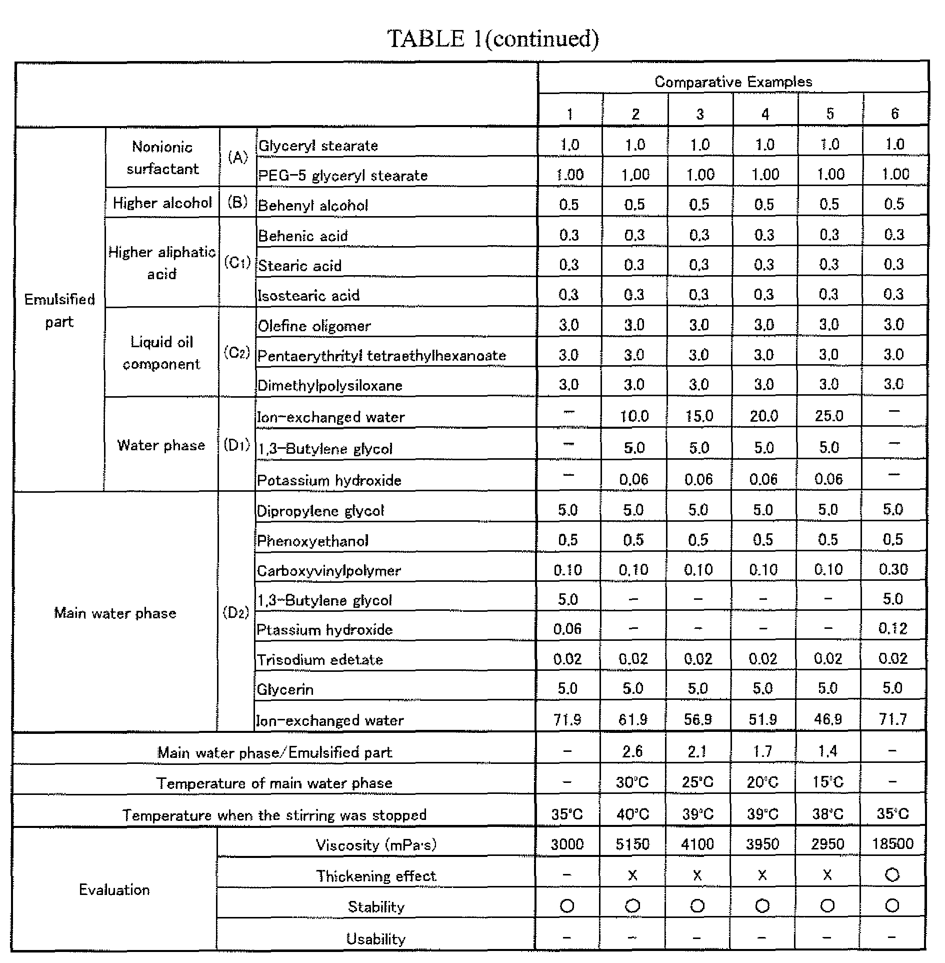

### Production Examples 1 to 4 and Comparative Examples 2 to 5

An O/W emulsion (emulsified part) was obtained by emulsifying (A), (B), (C₁), (C₂), and (D₁) listed in the table at 70 °C. While this emulsified part was being stirred, the main water phase (D₂) at a specified temperature was mixed to the emulsified part. The temperature of the mixture at the completion of the mixing of the main water phase was 40.2 °C or lower in all cases. When the specified temperature was reached, the stirring was stopped. Subsequently, the mixture was left to cool to room temperature to obtain an O/W emulsion composition.

### Comparative Examples 1 and 6

While (A), (B), (C₁), and (C₂) listed in the table were being stirred with a homogenizer, (D₁) and (D₂) were added to the mixture to carried out emulsification at 70 °C. The obtained W/O emulsion was cooled to 35 °C by passing it through an Onlator. Subsequently, it was left to cool to room temperature to obtain an O/W emulsion composition.

The results for the measurement of exothermic peaks of the emulsified part are shown in Fig. 1. The measurement was carried out with a DSC (Q-1000, TA Instruments, USA) by decreasing the temperature at a rate of 2 °C per minute from 70 °C to 30 °C. As a result, the peak temperature of the exothermic peak due to α-gel formation, for the emulsified part having the formulation of the table above, was 44.2 °C.

As is clear from the Table 1, it was found that, in Examples 1 to 4, an O/W cream that was highly viscous and excellent in thickening effect, stability, and usability could be obtained because the stirring was stopped at a higher temperature than the peak temperature (44.2 °C) in the temperature range of α-gel formation. On the other hand, in Comparative Examples 2 to 5, a high-viscosity O/W cream could not be obtained because the stirring was stopped at a lower temperature than the peak temperature.
In Comparative Example 1, wherein the conventional process with Onlator cooling was used, the viscosity of the composition was 3,000 mPa·s, and a high-viscosity O/W cream could not be obtained, although the formulation was the same as that of Example 1, Accordingly, when the conventional process is used, a highly viscous cream cannot be obtained unless a large amount of a thickener such as carboxyvinyl polymer is blended as in Comparative Example 6. When a large amount of a thickener is blended as in Comparative Example 6, the thickening effect and stability are good; however, the usability is very poor compared with Examples 1 to 4.

Furthermore, it was found from the results of Examples 1 to 4 that the smaller the blending quantity of the main water phase with respect to the blending quantity of the emulsified part, the lower main water phase temperature than room temperature might be necessary.
Accordingly, it was clarified that the preferable mass ratio of the main water phase and the emulsified part was 3: 1 to 1:1.

Thus, it was found that a cream could easily be produced with low energy, without the use of a cooling machine such as an Onlator, according to the production method of the high-viscosity O/W cream of the present invention, and that it was very economical. Furthermore, it was clarified that even when the formulation was the same as that for a low-viscosity cosmetic, a high-viscosity O/W cream could be produced by the production method of the present invention, without using a large amount of thickeners; thus a cream without a sticky-feeling of use could be produced.

Hereinafter, the present invention will be further explained by examples. However, the present invention is not limited by these examples. The quantities in the following formulations are expressed in mass %.

### Example 5

| Cream | Blending quantity (mass %) |
|---|---|
| (1) Ion-exchanged water | balance |
| (2) Glycerin | 5.0 |
| (3) Butylene glycol | 5.0 |
| (4) Polyethylene glycol 1500 | 2.0 |
| (5) Ethanol | 3.0 |
| (6) Phenoxyethanol | 0.3 |
| (7) Paraben | 0.1 |
| (8) Potassium hydroxide | 0.1 |
| (9) Trisodium edetate | 0.05 |
| (10) Carboxyvinyl polymer | 0.1 |
| (11) Xanthan gum | 0.1 |
| (12) Behenyl alcohol | 0.5 |
| (13) Behenic acid | 0.3 |
| (14) Stearic acid | 0.4 |
| (15) Isostearic acid | 0.3 |
| (16) Petrolatum | 2.0 |
| (17) Squalane | 3.0 |
| (18) Decamethylcyclopentasiloxane | 3.0 |
| (19) Dimethylpolysiloxane | 2.0 |
| (20) Cetyl 2-ethylhexanoate | 2.0 |
| (21) PEG-60 glyceryl isostearate | 1.0 |
| (22) PEG-5 glyceryl stearate | 1.0 |
| (23) Perfume | 0.1 |

### Production method:

The water phase (a portion of water phase) consisting of a portion of (1) to (3) (i.e., about 30 mass % of the total amount of (1) to (3)) and (8) was heated to 70 °C. The oil phase consisting of (12) to (23) was heated to 70 °C. The oil phase was gradually added to the water phase and emulsified with a homomixer to prepare an emulsified part. While the emulsified part was being stirred, the remaining water phase components (main water phase) of (1) to (7) and (9) to (11) at 25 °C were added and mixed into the emulsified part to obtain a cream. The viscosity of the cream was 40,000 mPa·s/30 °C. (peak temperature in the α-gel formation temperature range: 44 °C, temperature when the stirring for mixing was stopped: 46 °C)

### Example 6

| Hair cosmetic | Blending quantity (mass%) |
|---|---|
| (I)Liquid paraffin | 5.0 |
| (2)Petrolatum | 2.0 |
| (3)Dimethylpolysiloxane | 5.0 |
| (4)Cetanol | 4.0 |
| (5)Stearyl alcohol | 1.0 |
| (6)1,3-Butylene glycol | 10.0 |
| (7)Polyoxypropylene glyceryl ether | 2.0 |
| (8)Polyoxyethylene glyceryl isostearate | 2.0 |
| (9)Lipophilic glyceryl monostearate | 2.0 |
| (10)Polyquaternium-10 | 0.5 |
| (11)Purified water | balance |
| (12)Perfume | 0.1 |

### Production method:

The water phase (a portion of water phase) consisting of a portion of (6) and (11) (i.e., about 30 mass % of the total amount of (6) and (11)) was heated to 70 °C. The oil phase consisting of (1) to (5), (7) to (10) and (12) was heated to 70 °C. The oil phase was gradually added to the water phase and emulsified with a homomixer to prepare an emulsified part. While the emulsified part was being stirred, the remaining water phase components (main water phase) at 25 °C were added and mixed into the emulsified part to obtain a hair cosmetic. The viscosity of the hair cosmetic was 30,000 mPa·s/30 °C. (peak temperature in the α-gel formation temperature range: 48 °C, temperature when the stirring for mixing was stopped: 50 °C)

### Example 7

| Skin cleanser | Blending quantity (mass%) |
|---|---|
| (1)Ethanol | 15.0 |
| (2)Sorbitol solution | 10.0 |
| (3)Polyoxypropylene(9) diglyceryl ether | 4.0 |
| (4)Castor oil | 2.0 |
| (5)Isostearic acid | 2.0 |
| (6)Stearic acid | 7.0 |
| (7)Lauric acid | 6.0 |
| (8)Myristic acid | 11.0 |
| (9)Palmitic acid | 3.0 |
| (10)Sadium lauryl glycol carboxylate | 3.0 |
| (11)Sodium N-methyltaurate | 5.0 |
| (12)Sodium hydroxide | 4.0 |
| (13)Sodium chloride | 0.5 |
| (14)Chamomilla recutita extract | 0.1 |
| (15)Dibutyl hydroxytoluene | 0.1 |
| (16)Tetrasodium hydroxyethane diphosphonate (30%) | 0.1 |
| (17)Trisodium edetate | 0.1 |
| (18)4-tert-Butyl-4'-methoxydibenzoylmetane | 0.05 |
| (19)2-Ethylhexyl paramethoxycinnamate | 0.05 |
| (20)Mixture of sucrose and sorbitol | 15.0 |
| (21)Coloring agent | 0.5 |
| (22)Purified water | balance |
| (23)Perfume | 0.1 |

### Production method:

The water phase (a portion of water phase) consisting of a portion of (22) (i.e., about 50 mass % of the total amount of (22)), (12) and (13) was heated to 70 °C. The oil phase consisting of (3) to (11), (18), (19) and (23) was heated to 70 °C. The oil phase was gradually added to the water phase and emulsified with a homomixer to prepare an emulsified part. While the emulsified part was being stirred, the remaining water phase components (main water phase) at 25 °C were added and mixed into the emulsified part to obtain a skin cleanser. The viscosity of the skin cleanser was 25,000 mPa·s/30 °C. (peak temperature in the α-gel formation temperature range: 48°C, temperature when the stirring for mixing was stopped: 53 °C)

### Example 8

| Cream foundation | Blending quantity (mass%) |
|---|---|
| (1)Talc | 3.0 |
| (2)Titanium dioxide | 4.0 |
| (3)Red iron oxide | 0.5 |
| (4)Yellow iron oxide | 1.5 |
| (5)Black iron oxide | 0.1 |
| (6)Bentonite | 0.5 |
| (7)Polyoxyethylene sorbitan monostearate | 1.0 |
| (8)Triethanolamine | 1.5 |
| (9)Dipropylene glycol | 8.0 |
| (10)Ion-exchanged water | balance |
| (11)Behenic acid | 0.5 |
| (12)Stearyl alcohol | 0.4 |
| (13)Isohexadecyl alcohol | 6.0 |
| (14)Glyceryl monostearate | 2.0 |
| (15)Liquid lanolin | 2.0 |
| (16) Liquid paraffin | 6.0 |
| (17)Paraben | 0.1 |
| (18)Perfume | 0.05 |

### Production method:

The mixture of (8) and (9) in which (6) and (17) were dispersed was added to a portion of (10) and stirred at 70 °C. The oil phase consisting of (7), (11) to (16) and (18) was dissolved with heating at 70 °C. The oil phase was gradually added to the mixture and emulsified with a homomixer to prepare an emulsified part. While the emulsified part was being stirred, (10) at room temperature and sufficiently mixed and pulverized powder components of (1) to (5) were added and mixed into the emulsified part to obtain a cream foundation. The viscosity of the cream foundation was 15,000 mPa-s/30 °C. (peak temperature in the α-gel formation temperature range: 47 °C, temperature when the stirring for mixing was stopped: 49 °C)

## Claims

1. A production method of a high-viscosity O/W cream comprising the steps of:
emulsifying, at 70 °C or higher, an oil phase with a water phase to prepare an emulsified part that is an O/W emulsion, wherein
the oil phase comprises
(A) a nonionic surfactant,
(B) a linear higher alcohol that has 16 or more carbon atoms and can form an α-gel in water with the nonionic surfactant, and
(C) an oil component, and
the water phase comprises
(D) water;
cooling the emulsified part while the emulsified part is being stirred; and
stopping the stirring at the peak temperature or higher in a temperature range wherein the oil phase forms an α-gel in the water phase, but lower than 70 °C.

2. The method according to claim 1, comprising the steps of:
emulsifying, at 70 °C or higher, the oil phase with a portion of the water phase to prepare the emulsified part,
mixing the remaining water phase, which is a main water phase and at 20 to 40 °C, with the emulsified part while the emulsified part is being stirred, to cool the emulsified part; and
stopping the stirring at the peak temperature or higher in the temperature range wherein the oil phase forms the α-gel in the water phase, but lower than 70 °C.

3. The method according to claim 1 or 2, wherein the temperature when the stirring is stopped is 55 °C or lower.

4. The method according to any of claims 1 to 3, wherein the peak temperature in the temperature range wherein the oil phase forms the α-gel in the water phase is the exothermic peak, which is measured by differential scanning calorimetry DSC, of the emulsified part.

5. The method according to any of claims 1 to 4, wherein the viscosity of the high-viscosity O/W cream measured with a B-type viscometer at 30 °C is 8,000 mPa·s or higher.

6. The method according to any of claims 2 to 5, wherein the mass ratio of the main water phase and the emulsified part is 3:1 to 1:1.
